# EUROPEAN PATENT APPLICATION

(11) **EP 1 728 872 A1**
(43) Date of publication of application: **06.12.2006**
(21) Application number: 05076298.8
(22) Date of filing: 02.06.2005
(51) Int. Cl.: C12P 13/04, C07C 229/48

(54) **Biosynthetic production of (5S,6S)-6-amino-5-hydroxycyclohexa-1,3-diene-1-carboxylic acid (2,3-CHA)**

(71) Applicant: DSM IP Assets B.V., 6411 TE Heerlen (NL); DSM Biotech GmbH, 40474 Düsseldorf (DE); Forschungszentrum Jülich GmbH, 52425 Jülich (DE)
(72) Inventor: Wubbolts, Marcel Gerhardus, 6132 BM Sittard (NL); Bovenberg, Roelof Ary, 3062 DA Rotterdam (NL); Sprenger, Georg, 71522 Backnan-Maubach (DE); Bongaerts, Johannes Josef, 51379 Leverkusen (DE); Kozak, Stefan, 70178 Stuttgart (DE); Müller, Michael, 52425 Jülich (DE)
(74) Representative: van Tol-Koutstaal, Charlotte A.

(57) **Abstract**

The invention relates to a process for the biosynthetic production and recovery of [5S,6S]-6-amino-5-hydroxycyclohexa-1,3-diene-1-carboxylic acid) catalyzed, at increased enzyme activity, at least by an enzyme belonging to the class of aminodeoxyisochorismate (ADIC) synthases and an enzyme belonging to the class of isochorismatases, wherein
(a) the production is carried out fermentatively with a suitable carbon source as starting material in a chorismate overproducing host microorganism, and with an aminodeoxyisochorismate (ADIC) synthase enzyme selected from the group of such ADIC synthase enzymes that are lacking ADIC lyase activity, and
(b) the recovery is carried out by means of concentrating the supernatant of the fermentation to a 2,3-CHA concentration of at least 25 g/l, followed by crystallisation from the said concentrated supernatant.

In a second embodiment, the present invention relates to the use of [5S,6S]-6-amino-5-hydroxycyclohexa-1,3-diene-1-carboxylic acid (2,3-CHA) as a catalytically active product, in particular as a chiral catalyst.

Finally, the invention relates to the novel zinc complex of 2,3-CHA.

SEQUENCE LISTING

<110> DSM Biotech GmbH
Forschungszentrum Jülich GmbH
DSM IP Assets BV

<120> Biosynthetic production and recovery of [5S,6S]-6-amino-5-h
ydroxycyclohexa-1,3-diene-1-carboxylic acid (2,3-trans-CHA)

<130> 24443EPP0

<160> 2

<170> PatentIn version 3.1

<210> 1
<211> 36
<212> DNA
<213> Artificial sequence

<220>
<223> primer

<400> 1
cgcaacctga gctctggaga ccgtggcatg agcggc
36

<210> 2
<211> 36
<212> DNA
<213> Artificial sequence

<220>
<223> primer

<400> 2
catgggggat cctggctcag ggccgccgct cgacca
36

## Description

The present invention relates, in a first embodiment thereof, to a process for the biosynthetic production and recovery of [5*S*,6*S*]-6-amino-5-hydroxycyclohexa-1,3-diene-1-carboxylic acid (hereinafter also referred to as 2,3-trans-CHA, or even shorter as 2,3-CHA) catalyzed, at increased enzyme activity, at least by an enzyme belonging to the class of aminodeoxyisochorismate (ADIC) synthases and an enzyme belonging to the class of isochorismatases. In the literature [5S,6S]-6-amino-5-hydroxycyclohexa-1,3-diene-1-carboxylic acid sometimes is also referred to as *trans*-2,3-dihydro-3-hydroxy anthranilic acid (or DHHA). This compound is considered to be an important chiral building block, for instance, for the synthesis of various interesting pharmaceutical products such as phenazine antibiotics. For instance, see one or more of the following references: Floss, H. G., Nat. Prod. Rep. 1997, 14, p.433-452; Mavrodi, D.V. et al., J. Bacteriol. 1998, 180, p.2541-2548; Parsons, J.F. et al., Biochemistry 2003, 42, p.5684-5693; Dong, H. et al., J. Am. Chem. Soc. 2001, 123, p.2733-2742; Mahmud, T., Nat. Prod. Rep. 2003, 20, p. 137-160. It is a specific advantage of the present invention, that the supernatant in the production process for 2,3-CHA, and subsequently the 2,3-CHA produced and recovered, is substantially anthranilate-free. Since both anthranilate and 2,3-CHA are amino acids, separation of these substances is generally considered to be troublesome. In the process of the invention, 2,3-CHA is now obtained in high yields in a simple process.

In a second embodiment, the present invention relates to the use of [5*S*,6*S*]-6-amino-5-hydroxycyclohexa-1,3-diene-1-carboxylic acid (2,3-CHA) as a catalytically active product, in particular as a chiral catalyst. Finally, the invention relates to the novel zinc complex of 2,3-CHA.

A process for the biochemical synthesis of 2,3-CHA catalyzed, at increased enzyme activity (namely by using a T7 promoter), *inter alia,* by an enzyme belonging to the class of aminodeoxyisochorismate (ADIC) synthases and an enzyme belonging to the class of isochorismatases has been suggested by McDonald, M., et al., J. Am. Chem. Soc. 2001, 123, p.9459-9460 as part of their studies relating to biosynthetic pathways for phenazine-1-carboxylic acid. Scheme 1 of said article shows (in a reaction scheme linked to the primary shikimate pathway) that the enzymes PhzE (which in the article is referred to as a bacterial anthranilate synthase, but is known to consist of a part having ADIC synthase activity and a part having ADIC lyase activity; see below) and PhzD (a bacterial isochorismatase) may be involved in the last steps for arriving at DHHA from chorismic acid formed as an intermediate. However, as the authors have clearly shown in experiment no.1 of the said article, such mechanism is rather unlikely to occur because, at levels up to 5 mg/ml, chorismic acid is not a suitable substrate for the said reaction nor for the formation of ADIC. The fact, that McDonald *et* al. show that the substrate ADIC may lead to DHHA (= 2,3-CHA) accumulation, does not provide any teaching on how a 2,3-CHA production process starting from chorismate (or from products metabolisable to chorismate) could be achieved.

It is noted here, that in the literature, as is also being done in the aforementioned article of McDonald *et al.,* enzymes belonging to the class of aminodeoxyisochorismate (ADIC) synthases are sometimes also referred to as anthranilate synthases. Anthranilate synthases, however, usually are bifunctional enzymes performing both the ADIC synthase reaction and the ADIC lyase reaction. See, for instance, Morollo, A.A., et al., Proc. Natl. Acad. Sci. USA, 1993,90, p.9983-9987.

Besides the aforementioned (suggested, but apparently inefficient) biochemical route to 2,3-CHA also various chemical synthesis routes to 2,3-CHA are known, mostly leading to racemic mixtures thereof. Bunnage, M.E., et al. (Org. Letters, 2003, 5, p.239-242) have been able to produce (protected) + and - diastereoisomers of 2,3-CHA involving a PLE-assisted kinetic resolution of an intermediate in one of the otherwise chemical process steps. Total yield of the 2,3-CHA isomers in said 8-step process, however remained well below 10 % (calculated on the basis of the ethylacrylate starting material).

It is also to be noted here, that further processes leading to the formation of 2,3-CHA is known, for instance, from US-A-3,038,005; and from McCormick, J.R.D., et al., J. Am. Chem. Soc. 1961, 83, p.4104-4105, respectively 1962, 84, p.3711-3714. Although this further process indeed also involves some biochemical reaction (namely it starts by aerobically fermenting an aqueous nutrient medium with certain strains of the species *Streptomyces aureofaciens*)*,* the biochemical part of the synthesis route is rather undefined and it is even completely unknown whether any precursor is converted into 2,3-CHA in a biochemical step or not. As a matter of fact, it seems rather more likely, that the conversion into 2,3-CHA in said process only occurs during the work-up steps instead of in a biochemical reaction step: most of the formation of 2,3-CHA must have occurred chemically due to the severe conditions applied in the work-up phase. In said process ultimately very low concentrations of 2,3-CHA (about 6,5 g/I) and low yields (about 16 g from 10 I of fermentation medium) of crystalline 2,3-CHA product are obtained after troublesome recovery procedures requiring multiple recrystallisation steps.

Though *Streptomyces aureofaciens* strains are generally known to be difficult to handle because of safety aspects (according to German classification such organism is referred to as an "S2-organism") and because they require complex media, nevertheless also later research programs, aiming at the synthesis of heterocyclic polymers from 3-hydroxy anthranilic acid (a product that can be obtained from 2,3-CHA produced according to an improved method of the McCormick process using such *Streptomyces aureofaciens*)*.* Reference, for instance, can be made to US-A-5,340,913; in said document it is noted, that the yield of the McCormick procedure was not found to be reproducible. Accordingly, while starting from the same strain as McCormick, the inventors of US-A-5,340,913 developed - by modifications to the culture, growth and fermentation parameters, and media compositions - an improved method for synthesis of 2,3-CHA, which would allow for eliminating the need to purify 2,3-CHA before the dehydrogenation step to 3-hydroxy anthranilic acid. It can be seen that best yields of 2,3-CHA are in the range of 6-8 g/I before its dehydrogenation by Pd catalysis to 3-hydroxy anthranilic acid. However, the above references do not teach the production of 2,3-CHA in yields far above 8 g/I, nor of sufficiently pure 2,3-CHA, especially of 2,3-CHA substantially free of anthranilic acid. Pure 2,3-CHA (both diastereoisomerically pure and free of by-products, especially of anthranilate) will be necessary for conversion to other compounds than 3-hydroxy anthranilic acid (e.g. pharmaceutically active ingredients). The aforementioned improved production methods for 2,3-CHA using *Streptomyces aureofaciens* strains, thus, do not provide any other teaching on biosynthetic production of 2,3-CHA than the cited McCormick reference.

Accordingly, true biosynthesis of (and recovery in high amounts and high purity of) 2,3-CHA is, however, not known until now when starting from suitable carbon sources. There is, however, need for such truly biosynthetic process for the production of 2,3-CHA, by means of a simple and efficient process, also because generally such process is believed to be advantageous if it leads to an enantiomerically pure form of the compound. This will be most suitable as an intermediate for opening new routes to novel pharmaceutical derivatives thereof.

Disadvantages of the known process thus are: relatively low yields (and low purity) of crystalline 2,3-CHA recovered. Especially, serious contamination with anthranilate will be found, if no repeated recrystallisations and/or other purification steps are being performed. Accordingly, the relatively poor access to 2,3-CHA has so far limited the possibilities for development of further (for instance) pharmaceutically active substances derived from 2,3-CHA.

Accordingly, there is need for improved routes for producing 2,3-CHA biochemically by means of a simple and efficient process, the 2,3-CHA preferably being substantially anthranilate-free, without the aforementioned problems of the prior art.

The present inventors now surprisingly have found a process for the biosynthetic production and recovery of [5*S*,6*S*]-6-amino-5-hydroxycyclohexa-1,3-diene-1-carboxylic acid) catalyzed, at increased enzyme activity, at least by an enzyme belonging to the class of aminodeoxyisochorismate (ADIC) synthases and an enzyme belonging to the class of isochorismatases,
wherein the biosynthetic production of [5*S*,6*S*]-6-amino-5-hydroxycyclohexa-1,3-diene-1-carboxylic acid (2,3-CHA) is carried out fermentatively with a suitable carbon source as starting material in a chorismate overproducing host microorganism,
and wherein the enzyme belonging to the class of aminodeoxyisochorismate (ADIC) synthases is selected from the group of such ADIC synthase enzymes that are lacking ADIC lyase activity,
and wherein the recovery of 2,3-CHA is carried out by means of concentrating the supernatant of the fermentation to a 2,3-CHA concentration of at least 25 g/I, followed by crystallisation from the said concentrated supernatant.

As meant herein, the term "biosynthetic production" means any possible form of biochemical synthesis. Biochemical synthesis can be carried out either *in vivo* or *in vitro.* Generally, *in vivo* processes are processes carried out when using living cells (the term "living cells" thereby also including so-called resting cells); *in vitro* processes, on the other hand, usually are being carried out using cell lysates or (partly) purified enzymes. The biochemical synthesis as meant herein, however, also may be carried out using permeabilized cells; the differentiation between *in vivo* and *in vitro,* however, does not make much sense for processes being carried out with permeabilized cells. The process of the present invention is a fermentative process because the 2,3-CHA is produced from carbon source starting materials.

Carbon sources, which can suitably be used in the process according to the invention, are all carbon sources as can be used as precursors for the biosynthesis of chorismate. Examples of such carbon sources are oligosaccharides and disaccharides, for example maltose, sucrose, β-galactoside, melibiose, epimelibiose, galactinol, melibitol, galactosylglycerol and trehalose, hexoses, for example D-glucose, D-fructose, D-mannose, L-sorbose and D-galactose, glucose containing solutions or slurries, starch, carbon source containing solutions or slurries, amino sugars, for example N-acetyl-D-glucosamine and D-glucosamine, methylpentoses, for example L-fucose and L-rhamnose, pentoses and trioses, for example L-arabinose, D-arabinose, D-xylose, xylitol, D-lyxose, D-ribose, 2-deoxy-D-ribose and dihydroxyacetone, pentoses in nucleosides and deoxynucleosides, for example cytidine, deoxycytidine, adenosine, deoxyadenosine, uridine, xanthosine, thymidine (deoxyuridine), purine (adenine, hypoxanthine, guanine ribonucleoside), hexuronides, hexuronates and hexonates, for example D-gluconate and D-galactonate, phosphorylated sugars and carboxylates, for example hexose phosphates, and sn-glycerol 3-phosphate, dicarboxylates, for example succinate, fumarate and L-malate, tricarboxylic acids, polyols, for example D-mannitol, D-glucitol, D-sorbitol, galactitol, dulcitol, D-arabitol, ribitol and xylitol, glycerol, two-carbon compounds, for instance ethanol and acetate, fatty acids, glycolate and glyoxylate, but also methanol, edible oils, or mixtures of any of the above compounds.

Preferably the carbon source is selected from the group of glucose, glucose containing solutions or slurries, (m)ethanol, gluconate, pentoses, hexoses, starch and fatty acids. In particular, the carbon source used is glucose.

The biosynthetic production according to the invention can most suitably be carried out *in vivo.*

The term "at increased enzyme activity" as used in this patent application is intended to reflect the skilled man's understanding that such "increased activity" is always higher than the activity of the enzyme as naturally existing in the microorganism used under otherwise identical conditions. Accordingly, the reference level of enzyme activity is assessed in the situation naturally existing in the microorganism. It will be understood that such increased level of enzyme activity can be achieved by all means known to the skilled man, for instance by overexpression of the corresponding gene.

The process according to the invention is carried out in a chorismate overproducing host organism (or, as intended to be included within the meaning of the term "chorismate overproducing host organism", in permeabilized cells thereof).

Chorismate producing microorganisms generally are microorganisms wherein first shikimate is being formed via any of the known shikimate biosynthetic pathways. Further conversion of shikimate formed into chorismate takes place by means of phosphorylation, addition of a further pyruvate group and dephosphorylation. In fact, this full biosynthetic reaction sequence forms part of the aromatic amino acid pathway. Conversion of chorismate by means of an aminodeoxyiso-chorismate (ADIC) synthase (for instance: TrpE) then leads to the formation of anthranilate through the intermediate aminodeoxy-isochorismate (ADIC). Anthranilate, on its turn, can be converted into tryptophan via the tryptophan pathway. Moreover, conversion of chorismate into phenazine is also known to occur in various organisms. However, for many other organisms phenazine synthesis does not form part of the natural metabolism. For instance in *Escherichia coli,* 2,3-CHA is not known as a metabolite of any of the biochemical processes occurring in said organism.

It is, moreover, mentioned in an article of Laursen, J.B., et al. in Chem. Rev. 104, p. 1663-1685 (2004), entitled "Phenazine natural products: biosynthesis, synthetic analogues and biological activity", that phenazine natural products are isolated primarily from *Pseudomonas* and *Streptomyces,* and a few other genera from soil or marine habitats. Examples of such other species are: *Methanosarcina mazei* Gö1, *Pelagiobacter variabilis, Vibrio* strains, *Erwinia herbicola* (this is a strain also being named as *Pantoea agglomerans), Burkholderia phenazinium, Waksmania aerata,* and *Sorangium* species.

Chorismate overproducing host organisms are host organisms that, due to genetic modification of their basic metabolism, are capable of giving increased biosynthetic production of chorismate. Such host organisms, for instance (and preferably) have been engineered by a combination of the following measures: deletion of the *tyrA* and *pheA* genes, insertion of the *aroF*^{for} gene (feed-back-resistant DAHP), and/or overexpression of *aroFBL* genes. The skilled man can easily assess whether a host strain is a suitable chorismate overproducer, or not.

Further, according to the invention, the enzyme belonging to the class of aminodeoxyisochorismate (ADIC) synthases should be selected from the group of such ADIC synthase enzymes that are lacking ADIC lyase activity.

ADIC synthases lacking ADIC lyase activity are enzymes that generally show homology to anthranilate synthases (EC 4.1.3.27). The skilled man can easily, by simple screening methods, determine whether an ADIC synthase lacks ADIC lyase activity. For instance, screening for ADIC synthase activity of an enzyme can be done by expressing such enzyme and applying it in an enzyme assay for conversion of chorismate into ADIC and analysing the ADIC formed by means of High Performance Liquid Chromatography (HPLC) techniques. The skilled man then can easily establish whether ADIC lyase activity is missing in such enzyme having ADIC synthase activity, by expressing the enzyme in an *E. coli* mutant strain lacking the anthranilate synthase gene *trpE.* Such strain is incapable of growing on glucose mineral medium without addition of L-tryptophan. If, nevertheless, such strain, with the expressed enzyme to be checked for ADIC lyase activity, shows growth on glucose mineral medium without addition of L-tryptophan, ADIC lyase activity must be present.

ADIC synthases lacking ADIC lyase activity can also be obtained by selectively knocking-out any ADIC lyase activity from the genetic information of the host organism, if such ADIC lyase activity would be present therein. The skilled man will easily be able to determine which enzymes (and which genes coding for such enzymes) provide the ADIC lyase activity in the host strain. Accordingly, the skilled man will be able to selectively knock-out the ADIC lyase activity from the host organism to be used in the process of the present invention.

In the process of the invention, recovery of 2,3-CHA is carried out by means of concentrating the supernatant of the fermentation to a 2,3-CHA concentration of at least 25 g/l, followed by crystallisation from the said concentrated supernatant. This ensures the 2,3-CHA formed to be recovered in high yields from the supernatant of the fermentation. It will be clear that recovery of 2,3-CHA is most effectively if first all biomass is separated from the fermentation broth. The skilled man can easily apply any of the known methods for separating biomass from fermentation broth, for instance by using one or more suitable centrifuges or other equipment suitable for carrying out such separation. After the biomass has been separated from the fermentation broth, a cell-free supernatant is obtained.

Concentrating the supernatant, preferably the cell-free supernatant, can be done, for instance, by evaporating part of the aqueous solvent at increased temperature, and optionally at reduced pressure, to arrive at a 2,3-CHA concentrating of at least 25 g/I. Excessively high temperatures should be avoided during the concentration step, but no problems are encountered, if a cell-free supernatant is concentrated at a temperature in the range of from 50 to 80°C.

Preferably, the supernatant of the fermentation is concentrated to a 2,3-CHA concentration of at least 50 g/l.

Crystallisation from the concentrated supernatant of the fermentation, preferably the cell-free concentrated supernatant thereof, can be carried out by any method known to the skilled man, for instance by direct or indirect cooling, or by freeze-drying, or by evaporation of the solvent, optionally under conditions of reduced pressure, or by pervaporation. Adjustment of pH may also be applied. Generally, the pH during the crystallisation step will be in the range of from 5 to 8, more preferably in the range of from 5.6 to 7.3, and most preferably in the range of from 6 to 7.

More preferably, the crystallisation from the concentrated supernatant is carried out subsequent to precipitation and removal of proteins present in the said supernatant. Such precipitation and removal of proteins present in the concentrated supernatant can be carried out by any suitable method known to the skilled man. For instance, by means of centrifugation or filtration (for instance, by ultrafiltration) of the concentrated supernatant still containing the 2,3-CHA in completely dissolved form so that losses of 2,3-CHA during the protein removal step are avoided.

In view of the teachings of McDonald *et al.* and McCormick *et al.* (see above) it is completely unexpected that the process of the present invention leads to such high yields of 2,3-CHA that is substantially anthranilate-free, both in the supernatant as well as in the solid product recovered.

As meant herein, the term "substantially anthranilate-free" indicates, that the concentration of anthranilic acid in the 2,3-CHA crystallized and recovered is less than 0.5 % by weight, preferably less than 0.1 % by weight. In the supernatant of the biosynthetic production process of 2,3-CHA according to the invention, the concentration of anthranilic acid is generally less than 10 mg/l. The detection level of anthranilic acid is about 1 mg/l (detection by HPLC RP 8, 25 cm x 3 mm, 19.5 min, DAD 275 nm).

In the process according to the invention the biosynthetic production of 2,3-CHA is carried out fermentatively with a suitable carbon source as starting material in a chorismate overproducing host microorganism.

Preferably, the chorismate overproducing host microorganism is selected from the group of microorganisms from the group of genera consisting of *Bacillus, Corynebacterium, Escherichia, Pichia, Pseudomonas* and *Saccharomyces* strains.

More preferably, the biosynthetic production is carried out in a host microorganism selected from the group of species *Bacillus subtilis, Corynebacterium glutamicum, Escherichia coli, Pichia pastoris, Pseudomonas putida,* and *Saccharomyces cerevisiae.* Most preferably, the biosynthetic production is carried out in an *Escherichia coli* host microorganism.

Many enzymes belonging to the class of aminodeoxyisochorismate (ADIC) synthases are known. According to literature aminodeoxyisochorismate (ADIC) synthase generally consists of two subunits (α and β), capable of combining in the form of an αβ-dimer or α₂β₂-tetramer; the α- and β-subunits are coded for, respectively by *trpE* and *trpD* (in *E. coli* as well as in S. *typhimurium*)*.* The presence of said β-subunit(s) in such known aminodeoxyisochorismate (ADIC) synthases, however, also has anthranilate lyase activity. Morollo, A.A., et al., Proc. Natl. Acad. Sci. USA, 1993, 90, p.9983-9987, have demonstrated that such lyase activity in the β-subunits can be suppressed by an H398M mutation of the TrpE part, while leaving ADIC formation unaffected. The present inventors now surprisingly have been able to show that biosynthetic production of 2,3-CHA can favourably be carried out if catalyzed by means of the combined action, at increased enzyme activity, of (a) an aminodeoxyisochorismate (ADIC) synthase selected from the group of such ADIC synthase enzymes that are lacking ADIC lyase activity, and (b) an isochorismatase, in which process 2,3-CHA can be recovered in high yields and substantially free of anthranilate.

It is advantageous if the aminodeoxyisochorismate (ADIC) synthase enzyme lacking lyase activity is selected from the group of phenazine-specific anthranilate synthases.

The term "phenazine-specific anthranilate synthase" is often used in connection with phenazine biosynthesis. It can, for instance, be found in various annotations in protein databases, e.g. accession number AAC18904 for PhzE from *Pseudomonas fluorescens;* and accession number AAB00330 for PhzB from *Pseudomonas chlororaphis.*

Some examples of phenazine-specific anthranilate synthases are shown below in table 1, in which table also BLAST-P data are shown as to identity (i.e. homology) with the reference PhzE protein from *Pseudomonas aeruginosa* PAO1, as described by Mavrodi, D., et al., in J. Bacteriol. 183, p.6454-6465 (2001):

**Table 1**

| Accession number | Organism | Anthranilate synthase | Length | Identities |
|---|---|---|---|---|
| AAG05292 | *Pseudomonas aeruginosa* PA01 | Anthranilate/p-aminobenzoate synthases component I | 627 | 100% |
| --- | *Pseudomonas aeruginosa* ATCC 17933 | Anthranilate synthase | 627 | 99% |
| ZP_00137703 | *Pseudomonas aeruginosa* UCBPP-PA14 | Anthranilate/p-aminobenzoate synthases component I | 627 | 99% |
| ZP_00139567 | *Pseudomonas aeruginosa* UCBPP-PA14 | Anthranilate/p-aminobenzoate synthases component I | 627 | 98% |
| AAF17499 | *Pseudomonas chlororaphis* | Anthranilate synthase, phenazine-specific | 637 | 82% |
| AAB00330 | *Pseudomonas chlororaphis (Ps. aureofaciens* 30-84) | Anthranilate synthase, phenazine-specific | 637 | 82% |
| AAC18904 | *Pseudomonas fluorescens* | Anthranilate synthase, phenazine-specific | 637 | 82% |
| AAN40892 | *Pantoea agglomerans* | EhpC, phenazine biosynthesis | 634 | 50% |
| P00895 | *Escherichia coli* | Anthranilate synthase component I, TrpE | 520 | 27% |

It can be seen from this table, that homology with the PhzE protein from *Pseudomonas aeruginosa* is hardly relevant as criterium for determining whether an enzyme can suitably be used, according to the invention, as a phenazine-specific ADIC synthase enzyme lacking ADIC lyase activity. Already at a relatively low homology value enzymes are found having the same functionality as the reference enzyme. This means that functionality as such of the enzyme is more decisive than the said homology percentage. Nevertheless, and without specifically being bound to a lowest limit for the homology percentage with the reference PhzE protein from *Pseudomonas aeruginosa,* suitable phenazine-specific ADIC synthase enzymes lacking ADIC lyase activity for being applied in the process according to the invention will be at least about 25%, more preferably at least about 50%, even more preferably at least 70% or even at least 80%, and most preferably at least 90% homologous with the said reference enzyme.

Genes coding for PhzE are, for instance, known from an article of McDonald et al., JACS, 123, p.9459-9460 (2001). The phenazine genes have been identified and sequences by Mavrodi, D., et al., as described in J. Bacteriol. 180, p.2541-2548 (1998).

Genes coding for PhzE, and proteins having similar functionality as PhzE and being sufficiently homologous thereto, can, for instance, be obtained from any of the strains mentioned above in the article of Laursen, J.B., *et al.* Thus, preferably, the phenazine biosynthesis PhzE protein originates from a species from the group of genera consisting of *Pseudomonas, Pantoea, Streptomyces,* and *Erwinia.* In particular, the phenazine biosynthesis PhzE protein originates from a species selected from the group consisting of *Pseudomonas aeruginosa, Pseudomonas aureofaciens, Pseudomonas fluorescens, Pseudomonas chlororaphis,* and *Pantoea agglomerans* species. Most preferably, the phenazine biosynthesis PhzE protein originates from *Pseudomonas aeruginosa* PAO1 or from *Pseudomonas aeruginosa* ATCC 17933.

Preferably, the phenazine-specific anthranilate synthase is at least 70% homologous with PhzE from *Pseudomonas aeruginosa.* It is particularly preferred, that the phenazine-specific anthranilate synthase is PhzE from *Pseudomonas aeruginosa* PA01 or from *Pseudomonas aeruginosa* ATCC 17933.

It is further advantageous, if the enzyme belonging to the class of isochorismatases is selected from the group of phenazine-specific isochorismatases.

Also for these enzymes, the functionality as such of the enzyme is more decisive than the homology percentage in comparison to the reference enzyme PhzD from *Pseudomonas aeruginosa.* Nevertheless, and without specifically being bound to a lowest limit for the homology percentage with the reference PhzD protein from *Pseudomonas aeruginosa,* suitable phenazine-specific isochorismatases for being applied in the process according to the invention will be at least about 25%, more preferably at least about 50%, even more preferably at least 70% or even at least 80%, and most preferably at least 90% homologous with the said reference enzyme.

Genes coding for PhzD are, for instance, known from an article of McDonald et al., JACS, 123, p.9459-9460 (2001). The phenazine genes have been identified and sequences by Mavrodi, D., et al., as described in J. Bacteriol. 180, p.2541-2548 (1998).

Genes coding for PhzD, and proteins having similar functionality as PhzD and being sufficiently homologous thereto, can, for instance, be obtained from any of the strains mentioned above in the article of Laursen, J.B., *et al.* Thus, preferably, the phenazine biosynthesis PhzD protein originates from a species from the group of genera consisting of *Pseudomonas, Pantoea, Streptomyces,* and *Erwinia.* In particular, the phenazine biosynthesis PhzD protein originates from a species selected from the group consisting of *Pseudomonas aeruginosa, Pseudomonas aureofaciens, Pseudomonas fluorescens, Pseudomonas chlororaphis,* and *Pantoea agglomerans* species. Most preferably, the phenazine biosynthesis PhzD protein originates from *Pseudomonas aeruginosa* PAO1 or from *Pseudomonas aeruginosa* ATCC 17933.

Preferably, the isochorismatase is at least 70% homologous with PhzD from *Pseudomonas aeruginosa.* It is particularly preferred, that the phenazine-specific isochorismatase is PhzD from *Pseudomonas aeruginosa* PAO1 or from *Pseudomonas aeruginosa* ATCC 17933.

According to the present invention, the 2,3-CHA recovered by crystallisation from the cell-free supernatant, that has been concentrated to a 2,3-CHA concentration of at least 25 g/l, preferably of at least 50 g/l, optionally subsequent to precipitation and removal of proteins still present in the supernatant, is obtained in high purity, especially in high diastereoisomerical purity. Chemically, the 2,3-CHA recovered without any further purification steps will contain up to about 10 wt.% of co-precipitated salts. Removal of these salts will often be unnecessary, if the 2,3-CHA is subjected to further chemical stereospecific reactions. Nerevertheless, if so desired, further purification of the 2,3-CHA recovered may be achieved by washing (e.g. with cold water or methanol), and/or by re-crystallisation(s).

The present inventors have further found, that the 2,3-CHA prepared according to the method of the present invention can very suitably be used as a catalyst, and in particular as a chiral catalyst, for instance in the aldol condensation of 4-nitrobenzaldehyde with acetone in the presence of a Zn-complex with 2,3-CHA. 2,3-CHA is a chiral molecule. The enantiomeric excess of the chiral product obtained by chiral catalysis with 2,3-CHA, for instance the e.e. of the product [S]-1-p-nitrophenyl-2-hydroxybutane-3-one, is surprisingly much higher than in the case of the known chiral catalysis reaction using the Zn-complex with L-proline as ligand. Various general reviews and articles teach asymmetric catalysis with compounds such as L-proline. For instance, see List, B., et al., J. Am. Chem. Soc., Vol.122, p.2395-2396, 2000; Dalko, P.I. et al., Angew Chem. lnt. Ed. 40, p.33726-22748, 2001; List, B., Tetrahedron 58, p.5573-5590, 2002; Chandrasekhar, S., et al., Tetrahedron Lett., Vol.45, p.4581-4582, 2004; Berkessel, A., et al., Asymmetric Organocatalysis, VCH, Weinheim, 2004; Seayad, J., et al., Org. Biomol. Chem., Vol.3, p.719-724, 2005.

On the other hand it can be noted that chiral catalysis using α-amino acids - also other amino acids than L-proline are shown - is known, for instance, from two recent publications, namely of: Darbre, T., et al., Chem. Commun., 2003, p.1090-1091, showing catalysis by means of Zn-salts; and Amedjkouh, M., Tetrahedron: Asymmetry, Vo1.16, p.1411-1414, 2005, showing use in, for instance, asymmetric aldol reactions.

None of the abovementioned reviews and articles, however, provides any teaching regarding the use of β-amino acids and/or derivatives thereof for chiral catalysis. It may, however, be noted, that β-amino acids (e.g. β-alanine) indeed already have been described (e.g. by Prout, F.S., in J. Org. Chem., Vol. 18, p.928-933, 1953) as catalysts in the Knoevenagel reaction, a reaction that cannot be catalyzed by α-amino acids (such as α-alanine). But such disclosure cannot make the use of 2,3-CHA as a catalyst, and in particular as a chiral catalyst, obvious.

Accordingly and surprisingly, the present invention, in a second and independent embodiment, also relates to the use of [5S,6S]-6-amino-5-hydroxy-cyclohexa-1,3-diene-1-carboxylic acid (2,3-CHA), obtained by the process according to the first embodiment of the invention, as a catalytically active product, in particular as a chiral catalyst.

The present invention also relates to the novel Zn-complex of 2,3-CHA, characterized by the ¹H-NMR (D₂O) characteristics: δ = 4.11 (br s, 1 H), 4.39 (br s, 1 H), 6.22 (dd, J = 4.2 Hz, 9.5 Hz, 1 H), 6.34 (dd, J = 5.4 Hz, 9.5 Hz, 1 H), 6.96 (d, J = 5.4 Hz, 1 H) ppm.

The invention now will be elucidated by means of the following Examples (which, however, are not to be considered to limit the precise scope of the invention to the embodiments shown) and Comparative Examples.

### Experimental part

### Example 1: Construction of plasmid pC27

Standard molecular cloning techniques such as plasmid DNA isolation, gel electrophoresis, enzymatic restriction modification of nucleic acids, *E. coli* transformation etc. were performed as described by Sambrook *et al.,* 1989, "Molecular Cloning: a laboratory manual", Cold spring Harbor Laboratories, Cold Spring Harbor, NY. Synthetic oligonucleotides were obtained from MWG-Biotech AG (Ebersberg, Germany). DNA sequence analyses were performed by GATC Biotech AG (Konstanz, Germany) and AGOWA GmbH (Berlin, Germany).

As a host strain for 2,3-CHA production E. *coli* KB532 (*Δ*(*pheA-tyrA*), *ΔtyrR, aroF*^{fbr}, *thiA, hsdR17, endA1, supE44)* was chosen. KB532 is an L-tyrosine and L-phenylalanine auxotrophic strain. It lacks the genes for *tyrA* (chorismate mutase/prephenate dehydrogenase), *pheA* (chorismate mutase/prephenate dehydratase), and for the global regulator *tyrR,* and carries *aroF*^{fbr} encoding a feedback (L-tyrosine) resistant DAHP synthase.

As expression vector for the 2,3-CHA biosynthesis genes, plasmid pJF119EH was chosen; Fürste, et al. (1986, Gene, 48:119-131). The expression system uses the IPTG inducible tac promoter and carries the *lac* repressor (*lac*l^{q} gene), which keeps the expression of the cloned foreign gene in the absence of the inducer extremely low.

Genomic DNA from *Pseudomonas aeruginosa* ATCC 17933 was obtained from the American Type Culture Collection (ATCC), Manassas, VA, USA. A 2547 bp fragment comprising the open reading frames (ORF) for PA1902 and PA1903, encoding the phenazine biosynthesis proteins PhzD and PhzE was amplified by PCR from the chromosomal DNA from *Pseudomonas aeruginosa* ATCC 17933 (nucleotides 3857-6360 of accession number AE004616; amplified region nucleotides 3844-6364) using the following primers:
5'- CGCAACCTGAGCTCTGGAGACCGTGGCATGAGCGGC - 3'[SEQ ID: No. 1] (with *Sac*l recognition site underlined) and
5' - CATGGGGGATCCTGGCTCAGGGCCGCCGCTCGACCA - 3'[SEQ ID: No.2]
(with *Bam*HI recognition site underlined).

A list of all oligonucleotide sequences used in the context of the present application is presented in the SEQUENCE LISTING annexed hereto.

Correct size of the amplified fragment was confirmed by agarose gel electrophoresis and the fragment was purified from the gel. The fragment was restricted with the enzymes *Sac*l and *Bam*Hl to generate sticky ends. The plasmid pJF119EH was restricted with *Sac*l and *Bam*Hl. The two fragments were subsequently ligated and used for the transformation of chemically competent cells of *E. coli* DH5α (Invitrogen GmbH, Karlsruhe, Germany). The transformants were selected on LB agar plates containing 100 mg·l⁻¹ ampicillin. A plasmid showing the correct insert sequence (as confirmed by sequencing) was called pC27.

It is to be noted that the *phzD* and *phzE* genes occur twice in the genome of *Pseudomonas aeruginosa.* An exact copy can be found under accession number AE004838 nucleotides 2623-5126 (PA4213 and PA4214).

### Example 2: Fermentative production of 2,3-CHA

The 2,3-CHA production of *E. coli* KB532/pC27 from glucose was investigated in mineral medium. The precultivation medium consisted of MgSO₄·7H₂O (0.3 g·l⁻¹), CaCl₂·2H₂O (0.015 g·l⁻¹), KH₂PO₄ (3.0 g·l⁻¹), K₂HPO₄ (12.0 g·l⁻¹), NaCl (0.1 g·l⁻¹), (NH₄)₂SO₄ (5.0 g·l⁻¹), FeSO₄·7H₂O (0.075 g·l⁻¹), Na-citrate·3H₂O (1.0 g·l⁻¹), thiamine·HCl (0.0125 g·l⁻¹), L-tyrosine (0.05 g·l⁻¹), and L-phenylalanine (0.05 g·l⁻¹). Additional minerals were added in the form of a trace element solution (1.0 ml·l⁻¹), which trace element solution was composed of Al₂(SO₄)₃·18H₂O (2.0 g·l⁻¹), CoCl₂·6H₂O (0.75 g·l⁻¹), CuSO₄·5H₂O (2.5 g·l⁻¹), H₃BO₃ (0.5 g·l⁻¹), MnCl₂·4H₂O (20.0 g·l⁻¹) Na₂MoO₄·2H₂O (3.0 g·l⁻¹), NiSO₄·6H₂O (20 g·l⁻¹), ZnSO₄·7H₂O (15.0 g·l⁻¹). A glucose stock solution (500 g·l⁻¹) was autoclaved separately and added to the sterilized medium to a final concentration of 15 g·l⁻¹.

The stock culture of *E.* coli KB532/pC27 was stored at -80°C in Luria-Bertani (LB) medium containing 50% glycerol. 0.4 ml feedstock was used to inoculate 100 ml of precultivation medium containing ampicillin (100 mg·l⁻¹) in an 1 l shake flask and incubated at 33°C and 180 rpm for 16 hours.

The fermentation medium was the same as for precultivation except for the following changes: MgSO₄·7H₂O (0.9 g·l⁻¹), K₂HPO₄ was omitted, FeSO₄·7H₂O (0.1125 g·l⁻¹), Na-citrate·3H₂O (1.5 g·l⁻¹), thiamine·HCl (0.075 g·l⁻¹), L-tyrosine (0.15 g·l⁻¹), and L-phenylalanine (0.3 g·l⁻¹). Additional minerals were added in the form of a trace element solution (1.5 ml·l⁻¹).

Fed-batch cultivation was performed in a Labfors bioreactor (Infors, Einsbach, Switzerland) with 10% inoculation for 32.75 h; 2 l initial volume, cultivation temperature 33°C, pH 6,8. pH was controlled by 25 % ammonia titration. After 9.25 h a feed for L-tyrosine and L-phenylalanine (5 g·l⁻¹ L-tyrosine, 6 g·l⁻¹ L-phenylalanine dissolved in 1 N KOH; feed rate 7 g·h⁻¹) was started. At the same time a feed for glucose (500 g·l⁻¹; feed rate 22 g·h⁻¹) was started. After 9.25 h at an OD₆₂₀ₙₘ of 6,6 2,3-CHA production was induced by addition of 0.1 mM IPTG.

A sample of the culture supernatant was lyophilized and re-dissolved in D₂O. 600 MHz ¹H-NMR at 303 K showed the expected resonance spectrum.

The amount present was determined to be 11.9 g·l⁻¹ 2,3-CHA (0.56 g·g⁻¹ bdw). Besides said amount of 2,3-CHA, no chorismate, no ADIC, and no anthranilate were found to be present as determined in the supernatant by HPLC analysis.

The inventors performed the fermentative production of 2,3-CHA also at a fermentation scale of 300 I. In this larger scale experiment, even better results of 2,3-CHA production were obtained than the ones described above. 2,3-CHA was present (after 37 hours of fermentation) in a concentration of 15.7 g/I.

### Example 3: Isolation of 2,3-CHA from fermentation broth

Biomass was separated from the fermentation broth from the 300 l scale fermentation. Then the cell-free supernatant obtained was ultrafiltrated (spiral packed modul, 10 kD) at 10-20°C. Subsequently, the permeate was concentrated using a rotary evaporator (max. 60°C) until the 2,3-CHA concentration was about 70-80 g·l⁻¹. The concentrated liquid was kept at 4°C for crystallisation (4-5 days). The crystals were collected by filtration, washed with ice-cold methanol, and dried under vacuum (2 mbar) at room temperature. This procedure resulted in purities up to 90 % 2,3-CHA with inorganic salts as only impurities as determined by HPLC, NMR, and elementary analysis. The overall yield was around 60% if the mother liquid was recycled.

All sequences of nucleotides referred to in the experimental part are listed hereinafter, and will be submitted in electronic format (Patentln).

### Example 4: Catalysis with pure 2,3-CHA obtained according to the first embodiment of the invention (and Comparative Example A: same reaction with L-proline)

The inventors have demonstrated by means of a model reaction (Knoevenagel condensation analogous to the ones described in Cardillo, G., et al., Synth. Commun., Vol.33, p.1587-1594), and in comparison to the same reaction using L-proline as a catalyst, that 2,3-CHA itself can be used as a catalyst for said reaction.

### Comparative Example A

3-Methylbutyraldehyde (1.55 mmol; 172 µl of a liquid having a density of 0.801 g/ml) was dissolved in DMSO, and L-proline (0.2 mmol; 23.0 mg) was added. Then, after 5 minutes, dimethyl malonate (4 mmol; 459 µl of a liquid having a density of 1.156 g/ml) was added and the mixture was stirred over night at room temperature (r.t.). The reaction mixture was diluted with ethyl acetate and washed twice with water. The organic layer was dried over anhydrous Na₂SO₄. 297 mg of the condensation product, dimethyl 2-(3-methyl-butylidene)malonate, was obtained. Accordingly, the yield of the condensation product was 96%.

### Example 4

3-Methylbutyraldehyde (1.55 mmol; 172 µl of a liquid having a density of 0.801 g/ml) was dissolved in DMSO, and 2,3-CHA (0.2 mmol; 31.0 mg) was added. Then, after 5 minutes, dimethyl malonate (4 mmol; 459 µl of a liquid having a density of 1.156 g/ml) was added and the mixture was stirred over night at room temperature (r.t.). The reaction mixture was diluted with ethyl acetate and washed twice with water. The organic layer was dried over anhydrous Na₂SO₄. 243 mg of the condensation product, dimethyl 2-(3-methyl-butylidene)malonate, was obtained. Accordingly, the yield of the condensation product was 61 %.

### Example 5: Chiral catalysis with Zn-complex of pure 2,3-CHA obtained according to the first embodiment of the invention

### (and Comparative Example B: same reaction with L-proline)

The inventors have demonstrated by means of a model reaction (aldol condensation using zinc-complexes of 2,3-CHA analogous to the syntheses described by Darbre, T., et al. Chem. Commun., p.1090-1091, 2003), and in comparison to the same reaction using the zinc-complexes of L-proline as a catalyst, that zinc-complexes of 2,3-CHA show asymmetric catalytical activity, for instance in the formation of 4-hydroxy-4-(4-nitrophenyl)butan-2-one. Good e.e.'s can be obtained for the said reaction product.

No conversion at all was observed when the same reaction was performed using only zinc acetate as a catalyst.

### Comparative Example B

L-Proline (4.34 mmol; 499 mg) was dissolved in MeOH followed by addition of triethylamine (TEA; 0.6 ml of a liquid having a density of 0.73 g/ml). The mixture was stirred for 10 min at rt. Then, zinc acetate (2.17 mmol; 476 mg) was added to the mixture, and a colourless precipitate appeared immediately after the said addition. After stirring for 1 h the solid material was filtered and dried to get 532 mg (41%) of the zinc-(L-proline)₂ complex. This complex was characterized by the following ¹H-NMR (D₂O) characteristics: δ = 1.87 (br s, 3 H), 2.29 (br m, 1 H), 3.04 (br s, 1 H), 3.19 (br m, 1 H), 3.92 (br s, 1 H).

A small amount of the above zinc-(L-proline)₂ complex in water (0.05 mmol; 15 mg of the complex in 10 ml of water) was then added to a solution of 4-nitrobenzaldehyde (1.0 mmol; 151 mg) in 5 ml of acetone, and the reaction mixture was stirred at rt under inert conditions (N₂). After 4 h, the reaction mixture was evaporated and the residue was dissolved in chloroform. All non-soluble material was filtered. The filtrate was then rotatory evaporated to give 173 mg (83 % yield, 11 % e.e.) of 4-hydroxy-4-(4-nitrophenyl)butan-2-one. Determination of the e.e. was done by chiral phase HPLC (Chiralpak AS, Daicel).

This product was characterized by the following ¹H-NMR (D₂O) characteristics: δ = 2.23 (s, 3 H), 2.86 (m, 2 H), 3.61 (br s, 1 H, OH), 5.28 (dd, *J* = 7.7, 4.6 Hz, 1 H), 7.55 (d, *J* = 9.1 Hz, 2 H), 8.22 (d, *J* = 9.1 Hz, 2 H); respectively by the following ¹³C-NMR (D₂O) characteristics: δ = 30.9 (CH₃), 51.7 (CH₂), 69.1 (CH), 124.0 (2xCH), 126.6 (2xCH), 150.1 (2×C_{q}), 208.8 (C=O).

### Example 5

2,3-CHA (2.26 mmol; 350 mg) was dissolved in MeOH/H₂O followed by addition of triethylamine (TEA; 2.27 mmol; 312 µl of a liquid having a density of 0.73 g/ml). The mixture was stirred for 10 min at rt. Then, zinc acetate (1.13 mmol; 248 mg) was added to the mixture. After stirring for 1 h the solid material was filtered. From the filtrate a white solid precipitated after 3 h, which was separated and dried to give 244 mg (30% yield) of a zinc-(2,3-CHA)₂ complex. This complex was characterized by the following ¹H-NMR (D₂O) characteristics: δ = 4.11 (br s, 1 H), 4.39 (br s, 1 H), 6.22 (dd, J = 4.2 Hz, 9.5 Hz, 1 H), 6.34 (dd, J = 5.4 Hz, 9.5 Hz, 1 H), 6.96 (d, J = 5.4 Hz, 1 H) ppm..

A small amount of the above zinc-(2,3-CHA)₂ complex in water (0.05 mmol; 18.8 mg of the complex in 10 ml of water) was then added to a solution of 4-nitrobenzaldehyde (1.0 mmol; 151 mg) in 5 ml of acetone, and the reaction mixture was stirred at rt under inert conditions (N₂). After 70 h, the reaction mixture was evaporated and the residue was dissolved in chloroform. The non-soluble material was filtered. The filtrate was then rotatory evaporated to give 184 mg (88 % product, 26 % e.e.) of 4-hydroxy-4-(4-nitro-phenyl)butan-2-one. Determination of the e.e. was done by chiral phase HPLC (Chiralpak AS, Daicel).

It is to be noted, that the e.e. of this product showed a dependence of reaction time and conversion: after 43 h of reaction time and at about 22% conversion, the product even was very highly enantioenriched (88 % e.e.).

Clearly, excellent e.e.'s of the aldol condensation products can be obtained according to the second embodiment of the present invention.

## Claims

1. Process for the biosynthetic production and recovery of [5S,6S]-6-amino-5-hydroxycyclohexa-1,3-diene-1-carboxylic acid) catalyzed, at increased enzyme activity, at least by an enzyme belonging to the class of aminodeoxyisochorismate (ADIC) synthases and an enzyme belonging to the class of isochorismatases,
wherein the biosynthetic production of [5*S*,6*S*]-6-amino-5-hydroxycyclohexa-1,3-diene-1-carboxylic acid (2,3-CHA) is carried out fermentatively with a suitable carbon source as starting material in a chorismate overproducing host microorganism,
and wherein the enzyme belonging to the class of aminodeoxyisochorismate (ADIC) synthases is selected from the group of such ADIC synthase enzymes that are lacking ADIC lyase activity,
and wherein the recovery of 2,3-CHA is carried out by means of concentrating the supernatant of the fermentation to a 2,3-CHA concentration of at least 25 g/l, followed by crystallisation from the said concentrated supernatant.

2. Process according to claim 1,
**characterized in that**
the supernatant of the fermentation is concentrated to a 2,3-CHA concentration of at least 50 g/l.

3. Process according to claim 1 or 2,
the crystallisation from the concentrated supernatant is carried out subsequent to precipitation and removal of proteins present in the said supernatant.

4. Process according to any of claims 1 to 3,
**characterized in that**
the chorismate overproducing host microorganism is selected from the group of microorganisms from the group of genera consisting of *Bacillus, Corynebacterium, Escherichia, Pichia, Pseudomonas* and *Saccharomyces* strains.

5. Process according to claim 4,
**characterized in that**
the biosynthetic production is carried out in a host microorganism selected from the group of species *Bacillus subtilis, Corynebacterium glutamicum, Escherichia coli, Pichia pastoris, Pseudomonas putida* and *Saccharomyces cerevisiae.*

6. Process according to claim 5,
**characterized in that**
the biosynthetic production is carried out in an *Escherichia coli* host microorganism.

7. Process according to any of claims 1 to 6,
**characterized in that**
the aminodeoxyisochorismate (ADIC) synthase enzyme lacking lyase activity is selected from the group of phenazine-specific anthranilate synthases.

8. Process according to claim 7,
**characterized in that**
the phenazine-specific anthranilate synthase is at least 70% homologous with PhzE from *Pseudomonas aeruginosa.*

9. Process according to claim 8,
**characterized in that**
the phenazine-specific anthranilate synthase is PhzE from *Pseudomonas aeruginosa* PAO1 or from *Pseudomonas aeruginosa* ATCC 17933.

10. Process according to any of claims 1 to 9,
**characterized in that**
the enzyme belonging to the class of isochorismatases is selected from the group of phenazine-specific isochorismatases.

11. Process according to claim 10,
**characterized in that**
the enzyme belonging to the class of isochorismatases is at least 70% homologous with PhzD from *Pseudomonas aeruginosa.*

12. Process according to claim 11,
**characterized in that**
the phenazine-specific isochorismatase is PhzD from *Pseudomonas aeruginosa.*

13. Use of [5*S*,6*S*]-6-amino-5-hydroxycyclohexa-1,3-diene-1-carboxylic acid (2,3-CHA), obtained by the process according any of claims 1-12, as a catalytically active product, in particular as a chiral catalyst.

14. Zn-complex of 2,3-CHA, **characterized by** the ¹H-NMR (D₂O) characteristics: δ = 4.11 (br s, 1 H), 4.39 (br s, 1 H), 6.22 (dd, J = 4.2 Hz, 9.5 Hz, 1 H), 6.34 (dd, J = 5.4 Hz, 9.5 Hz, 1 H), 6.96 (d, J = 5.4 Hz, 1 H) ppm.
